(19) Europäisches Patentamt
European Patent Office
Office européen des brevets

(11) Veröffentlichungsnummer: **0 022 185**
**B1**

(12) # EUROPÄISCHE PATENTSCHRIFT

(45) Veröffentlichungstag der Patentschrift: **22.12.82**

(51) Int. Cl.³: **C 07 C 53/48**, C 07 C 51/62

(21) Anmeldenummer: **80103270.7**

(22) Anmeldetag: **12.06.80**

(54) **Verfahren zur Herstellung von Chloracetylchlorid.**

(30) Priorität: **06.07.79 DE 2927353**

(43) Veröffentlichungstag der Anmeldung: **14.01.81 Patentblatt 81/2**

(45) Bekanntmachung des Hinweises auf die Patenterteilung: **22.12.82 Patentblatt 82/51**

(84) Benannte Vertragsstaaten: **BE CH DE FR GB IT LI NL**

(56) Entgegenhaltungen:
**DE-A-2 263 580**
**DE-B-1 804 436**

**JOURNAL OF THE AMERICAN CHEMICAL SOCIETY, Bd. 60, 1938, Columbus, Ohio, USA, H.C. BROWN «A Convenient Preparation of Volatile Acid Chlorides», S. 1325 bis 1327**

**JOURNAL OF THE AMERICAN CHEMICAL SOCIETY, Bd. 42, 1920, Columbus, Ohio, USA, R. ADAMS *et al.* «The Use of Oxalyl Chloride and Bromide for Producing Acid Chlorides, Acid Bromides or Acid Anhydrides», S. 599 bis 611**

(73) Patentinhaber: **HOECHST AKTIENGESELLSCHAFT, Postfach 80 03 20, D-6230 Frankfurt/Main 80 (DE)**

(72) Erfinder: **Ohorodnik, Alexander, Dr., Kastanienweg 24, D-5042 Erftstadt (DE)**
Erfinder: **Neumaier, Hubert, Dr., Breslauer Strasse 76, D-5040 Brühl (DE)**
Erfinder: **Gehrmann, Klaus, Dr., Geschwister-Scholl-Strasse 32, D-5042 Erftstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Chloracetylchlorid

Gegenstand der Erfindung ist ein Verfahren zur Herstellung von Chloracetylchlorid, welches frei von höher chlorierten Nebenprodukten ist, und zu dessen Herstellung Acetylchlorid als Ausgangsprodukt verwendet wird.

Acetylchlorid fällt in der Technik vielfach als Nebenprodukt, beispielsweise bei der Chlorierung von Essigsäure in Gegenwart von Essigsäureanhydrid unter Bildung von Monochloressigsäure oder bei der Herstellung von 2,5-Dioxo-1-oxaphospholan entsprechend dem Verfahren der DE-PS Nr. 2531238 an oder kann auch als reines Produkt aus Essigsäureanhydrid und Chlorwasserstoff gewonnen werden.

Die Überführung des in der Technik wenig begehrten Acetylchlorids in das begehrtere Chloracetylchlorid bereitet erhebliche Schwierigkeiten, die im wesentlichen daraus resultieren, dass bei allen bisher bekannten Verfahren zur Herstellung von Chloracetylchlorid durch Chlorierung von Acetylchlorid Nebenprodukte, vor allem Dichloracetylchlorid, gebildet werden, die weder durch Destillation noch auf andere Weise aus dem Chloracetylchlorid entfernt werden können. Aus der Fachliteratur ist es bekannt, vorgenannte Nebenproduktbildung durch Einsatz von Katalysatoren zwar zu reduzieren, jedoch nicht gänzlich auszuschliessen. So wird beispielsweise in der deutschen Offenlegungsschrift Nr. 2263580 vorgeschlagen, die Chlorierung von Acetylchlorid mit Chlor in Gegenwart von konz. Schwefelsäure als Katalysator durchzuführen, während nach der Arbeitsweise der DE-OS Nr. 2729911 als Chlorierungskatalysator Chlorsulfonsäure eingesetzt wird. Durch die genannten Massnahmen kann man zwar den Gehalt an Dichloracetylchlorid, bezogen auf Chloracetylchorid, auf 1,5-0,5 Gew.%, reduzieren, jedoch bereitet die nachträgliche Entfernung des Katalysators beträchtliche Schwierigkeiten. Dieser Sachverhalt wird in der DE-OS Nr. 2729911 eingehend erörtert. Aber auch das in der DE-OS Nr. 2729911 beschriebene Verfahren stellt keine befriedigende technische Lösung dar, zumal der Gehalt an Dichloracetylchlorid gegenüber dem bisherigen Stand der Technik nur reduziert wird, andererseits höher siedende Nebenprodukte entstehen und auch das problem der Katalysatorabtrennung bestehen bleibt.

Ein weiteres Verfahren zur Herstellung von Monochloracetylchlorid ist der DE-AS Nr. 1804436 zu entnehmen und besteht in der Umsetzung von Monochloressigsäure mit Phosgen. Der Umgang mit dem hochgiftigen Phosgen ist mit erheblichen Risiken belastet, so dass man von dieser Arbeitsweise im technischen Massstab absieht.

Schliesslich offenbaren die Journale «J. Am. Chem. Soc.» 60 (1938), S. 1325-1327, und 42 (1920), S. 599-611, Arbeitsweisen zur Herstellung von Chloracetylchlorid, wobei Monochloressigsäure mit Benzoyl- bzw. Oxalylchlorid unter Bildung von Chloracetylchlorid und Benzolsäure bzw. Oxalsäure umgesetzt wird. Vorgenannte Chlorierungsmittel in Form von Benzoyl- bzw. Oxalylchlorid stehen erfindungsgemäss nicht zur Diskussion, da die Erfindung zwingend den Einsatz des als Nebenprodukt bei der Chlorierung von Essigsäure zu Monochloressigsäure anfallenden Acetylchlorids als Chlorierungsmittel vorschreibt, um das Nebenmprodukt durch Umwandlung einer technischen Verwertbarkeit zuzuführen. Acetylchlorid stellt ausserdem im Gegensatz zu Benzoylchlorid eine tiefsiedende Verbindung dar und erscheint deshalb aufgrund seiner Flüchtigkeit als Chlorierungsmittel nicht geeignet.

Es wurde nunmehr gefunden, dass man die bei den bekannten Verfahren zur Herstellung von Chloracetylchlorid auftretenden Schwierigkeiten vollständig vermeiden kann, wenn man das Acetylchlorid mit Monochloressigsäure gemäss folgender Gleichung

$$H_3C{-}COCl + ClCH_2{-}COOH \underset{}{\overset{HCl}{\rightleftharpoons}} ClCH_2{-}COCl + CH_3{-}COOH$$

umsetzt.

Um die Bildung von Säureanhydriden bei vorstehender Umsetzung zu vermeiden, ist es wichtig, die Reaktion in Anwesenheit von Chlorwasserstoff durchzuführen. Die Reaktion gemäss obiger Gleichung basiert auf einem sich bei höherer Temperatur schnell einstellenden Gleichgewicht, welches, experimentell bestimmt, bei etwa 65 mol% Acetylchlorid und 35 mol% Chloracetylchlorid liegt. Man kann die Gleichgewichtslage zugunsten der Bildung von Chloracetylchlorid beeinflussen, indem man dafür sorgt, dass im Reaktionsgemisch stets ein Überschuss an Monochloressigsäure gegenüber dem eingesetzten Acetylchlorid vorhanden ist. Dies kann bei einer kontinuierlichen Verfahrensweise beispielsweise durch geeignete Einbauten im Reaktor bewirkt werden.

Gegenstand der Erfindung ist somit ein Verfahren zur Herstellung von Chloracetylchlorid durch Chlorierung von Acetylchlorid, welches dadurch gekennzeichnet ist, dass man dampfförmiges Acetylchlorid und verflüssigte Monochloressigsäure in stöchiometrischen Mengen oder im molaren Überschuss von Monochloressigsäure in einer auf 110-180°C beheizten Reaktionszone in Gegenwart eines Chlorwasserstoffstromes im Gegenstrom unter Bildung von Chloracetylchlorid umsetzt, wobei das Acetylchlorid die Reaktionszone von unten nach oben und die Monochloressigsäure die Reaktionszone in umgekehrter Richtung durchströmt, und dass man aus dem Reaktionsgemisch ein Gemisch aus Chloracetylchlorid und nicht umgesetztem Acetylchlorid abtreibt und aus dem anfallenden Gemisch der Säurechloride Chloracetylchlorid durch Destillation des Gemisches abtrennt.

Das Molverhältnis von Acetylchlorid und Monochloressigsäure kann im Bereich von 1:1-25

variieren. Bei der erfindungsgemäss bevorzugten kontinuierlichen Verfahrensweise wird durch die Einbauten im Reaktor ein vielfacher molarer Überschuss an Monochloressigsäure in der Reaktionszone bewirkt. Bei einer diskontinuierlichen Arbeitsweise beträgt das Molverhältnis vorzugsweise 1:10-15.

Die in die Reaktionszone eingeleitete verflüssigte Monochloressigsäure besitzt im allgemeinen eine Temperatur von etwa 70 bis 120° C, während die Reaktionszone selbst vorzugsweise auf eine Temperatur von 120 bis 130° C beheitz ist.

Nach einer weiteren bevorzugten Ausführungsform des Verfahrens der Erfindung setzt man Acetylchlorid und Monochloressigsäure in der beheizten Reaktionszone im Gegenstrom um, wobei das Acetylchlorid die Reaktionszone von unten nach oben und die Monochloressigsäure die Reaktionszone in umgekehrter Richtung durchströmt. Vorteilhafterweise führt man zusammen mit den Acetylchloriddämpfen den Chlorwasserstoff der Reaktionszone zu, wobei man pro mol Acetylchlorid 0,5 bis 3 mol Chlorwasserstoff einsetzt.

Das erfindungsgemässe Verfahren kann z.B. so durchgeführt werden, dass man in ein senkrecht stehendes, auf 110-180° C beheiztes Reaktionsrohr mit Einbauten, beispielsweise in Form einer Glockenbodenkolonne, von oben flüssige Monochloressigsäure mit einer Temperatur von 100° C und von unten dampfförmiges Acetylchlorid sowie einen konstanten Chlorwasserstoffstrom zur Verhinderung der Bildung von Säureanhydriden kontinuierlich einleitet, wobei das Acetylchlorid auf den einzelnen Böden stets mit überschüssiger Monochloressigsäure in Berührung kommt. Das Molverhältnis von Monochloressigsäure zu Acetylchlorid beträgt mindestens 1:1. Es kann aber ein vielfacher Überschuss an Monochloressigsäure angewandt werden.

In einer am Kopf des Reaktors angebrachten Destillationskolonne wird unmittelbar ein Säurechloridgemisch, bestehend aus Chloracetylchlorid und nichtumgesetztem Acetylchlorid, von dem höher siedenden Säuregemisch, bestehend aus Chloressigsäure und Essigsäure, abgetrennt und kontinuierlich abgezogen. Aus dem so erhaltenen, säurefreien Säurechloridgemisch kann Chloracetylchlorid ohne Schwierigkeiten durch Destillation des Gemisches rein gewonnen werden, während das hierbei anfallende Acetylchlorid in den Reaktor zurückgeführt wird.

Der Reinheitsgrad des erhaltenen Chloracetylchlorids hängt zum Teil vom Reinheitsgrad der eingesetzten Monochloressigsäure ab. Überraschenderweise wurde festgestellt, dass auch bei Verwendung von technischer Monochloressigsäure mit einem Gehalt von etwa 0,4-0,6 Gew.% Dichloressigsäure ein Chloracetylchlorid erhalten wird, das frei von Dichloracetylchlorid ist. Das am Fuss des Reaktors anfallende Säuregemisch kann zur Herstellung von Monochloressigsäure eingesetzt werden.

Der wesentliche Vorteil des erfindungsgemässen Verfahrens besteht darin, dass ausser dem Verfahrensprodukt keine unerwünschten Nebenprodukte erhalten werden, und dass die Herstellung von Chloracetylchlorid einen geringen apparativen Aufwand erfordert.

Die folgenden Beispiele dienen der näheren Erläuterung des erfindungsgemässen Verfahrens:

*Beispiel 1*

In den oberen Teil einer auf 120° C beheizten Glokkenbodenkolonne wurden stündlich 189 g oder 2 mol Monochloressigsäure mit einer Temperatur von 100° C und einem Dichloressigsäuregehalt von 0,55 Gew.% eingeleitet. Gleichzeitig wurden in den unteren Teil der Kolonne stündlich 78,5 g oder 1 mol dampfförmiges Acetylchlorid sowie zur Verhinderung der Bildung von Säureanhydriden 30-40 l/h Chlorwasserstoffgas eingeleitet. Über eine am Kopf der Glockenbodenkolonne aufgesetzte kurze Kolonne wurde bei einem Rücklaufverhältnis von etwa 1:1 ein Gemisch aus Acetylchlorid und Chloracetylchlorid abgezogen. Aus dem ebenfalls über Kopf abströmenden Chlorwasserstoffstrom wurde mitgeführtes Acetylchlorid mittels einer Kühlfalle kondensiert. Innerhalb von 5 h wurden der Kolonne und Kühlfalle 395 g eines Gemisches entnommen, welches 30 Gew.% oder 118,5 g Chloracetylchlorid und 70 Gew.% oder 276,5 g Acetylchlorid enthielt.

Die Ausbeute an Chloracetylchlorid betrug 70,9%, bezogen auf umgesetztes Acetylchlorid, wobei das Chloracetylchlorid weniger als 0,1 Gew.% Dichloracetylchlorid enthielt.

Das nach Reindestillation des Säurechloridgemisches erhaltene Acetylchlorid wurde in den Reaktor zurückgeleitet, während das am Fuss des Reaktors anfallende Gemisch aus Monochloressigsäure und Essigsäure zur Herstellung von Monochloressigsäure verwendet wurde.

*Beispiel 2*

Es wurde analog Beispiel 1 verfahren, wobei jedoch stündlich 157 g oder 2 mol Acetylchlorid eingesetzt wurden. Innerhalb von 5 h wurden der Kolonne und Kühlfalle 809 g eines Gemisches entnommen, welches 12 Gew.% oder 97 g Chloracetylchlorid und 88 Gew.% oder 712 g Acetylchlorid enthielt.

Die Ausbeute an Chloracetylchlorid betrug 83,5%, bezogen auf umgesetztes Acetylchlorid, wobei das Chloracetylchlorid weniger als 0,1 Gew.% Dichloracetylchlorid enthielt.

*Beispiel 3*

Es wurde analog Beispiel 1 verfahren, wobei jedoch stündlich 94,5 g oder 1 mol Monochloressigsäure und 78,5 g oder 1 mol Acetylchlorid eingesetzt wurden. Innerhalb von 7 h wurden der Kolonne und Kühlfalle 571,1 g eines Gemisches entnommen, welches 12,2 Gew.% Chloracetylchlorid und 87,7 Gew.% Acetylchlorid enthielt.

Die Ausbeute an Chloracetylchlorid betrug 100%, bezogen auf umgesetztes Acetylchlorid, wobei das Chloracetylchlorid weniger als 0,1 Gew.% Dichloracetylchlorid enthielt.

**Patentanspruch**

Verfahren zur Herstellung von Chloracetylchlorid durch Chlorierung von Acetylchlorid, dadurch gekennzeichnet, dass man dampfförmiges Acetylchlorid und verflüssigte Monochloressigsäure in stöchiometrischen Mengen oder im molaren Überschuss von Monochloressigsäure in einer auf 110-180° C beheizten Reaktionszone in Gegenwart eines Chlorwasserstoffstromes im Gegenstrom unter Bildung von Chloracetylchlorid umsetzt, wobei das Acetylchlorid die Reaktionszone von unten nach oben und die Monochloressigsäure die Reaktionszone in umgekehrter Richtung durchströmt, und dass man aus dem Reaktionsgemisch ein Gemisch aus Chloracetylchlorid und nicht umgesetztem Acetylchlorid abtreibt und aus dem anfallenden Gemisch der Säurechloride Chloracetylchlorid durch Destillation des Gemisches abtrennt.

**Claim**

Process for making chloroacetyl chloride by subjecting acetyl chloride to chlorination, characterized in that acetylchloride in vapor form and liquefied monochloroacetic acid are reacted countercurently with respect to one another, in stoechiometric proportions or with the use of a molar excess of monochloroacetic acid inside a reaction zone heated to 110-180° C and in the presence of a stream of hydrogen chloride with the resultant formation of chloroacetyl chloride, the acetyl chloride flowing upwardly and the monochloroacetic acid flowing downwardly, through the reaction zone, in that a mixture formed of chloroacetyl chloride and unreacted acetyl chloride is expelled from the reaction mixture, and in that chloroacetyl chloride is separated from the resulting mixture of acid chlorides by subjecting the mixture to distillation.

**Revendication**

Procédé de préparation du chlorure de chloroacétyle par chloration de chlorure d'acétyle, caractérisé en ce que l'on fait réagir à contre-courant du chlorure d'acétyle gazeux et de l'acide monochloroacétique liquéfié, en proportions stœchiométriques ou avec un excès molaire d'acide monochloroacétique, dans une zone de réaction chauffée à 110-180° C, en présence d'un courant d'acide chlorhydrique avec formation de chlorure de chloroacétyle, le chlorure d'acétyle traversant la zone de réaction de bas en haut et l'acide monochloroacétique en sens inverse, en ce que l'on chasse du mélange réactionnel un mélange de chlorure de chloroacétyle et de chlorure d'acétyle n'ayant pas réagi, et en ce que l'on sépare du mélange des chlorures d'acides le chlorure de chloroacétyle par distillation du mélange.